# EUROPÄISCHE PATENTSCHRIFT

(11) **EP 1 102 536 B1**
(45) Veröffentlichungstag und Bekanntmachung des Hinweises auf die Patenterteilung: **02.05.2003**
(21) Anmeldenummer: 99942804.8
(22) Anmeldetag: 30.07.1999
(51) Int. Cl.: A01N 43/82, A61K 7/06, A61K 7/48, A61K 7/16

(54) **Verwendung von Oxathiazolonen als Mikrobizide**
Use of oxathiazolones as microbicides
Utilisation d'oxathiozalones comme microbicides

(30) Priorität: 04.08.1998 EP 98810749
(43) Veröffentlichungstag der Anmeldung: 30.05.2001
(73) Patentinhaber: Ciba Specialty Chemicals Holding Inc., 4057 Basel (CH)
(72) Erfinder: HÖLZL, Werner, F-68440 Eschentzwiller (FR); SCHNYDER, Marcel, CH-4127 Birsfelden (CH)
(86) Internationale Anmeldenummer: EP9905449
(87) Internationale Veröffentlichungsnummer: WO00007446

(56) Entgegenhaltungen:
- GB-A- 1 079 348
- SENNING ET AL.: "Reaktionen von Trichlormethansulfenylchlorid mit Stickstoffverbindungen IV. Substituenteneffekte in 5-Aryl-1,3,4-oxanthiazol-2-onen" ACTA CHEM. SCAND:, Bd. 27, Nr. 6, 1973, Seiten 2161-2170, XP002090136

## Beschreibung

Die vorliegende Erfindung betrifft die Verwendung von Oxathiazolonen zur antimikrobiellen Behandlung von Oberflächen, ausgewählt aus Haut, Schleimhäuten, Haaren und textilen Fasermaterialien.

Die GB-1.079.348 offenbart Oxathiazolonderivate, die den Verbindungen der Formel (1) in der vorliegenden Anmeldung entsprechen. Die Verbindungen werden als Hilfsprodukte zur Herstellung von synthetischen Harzen verwendet sowie als Fungizide.

Die erfindungsgemäss eingesetzten Oxathiazolone entsprechen der Formel 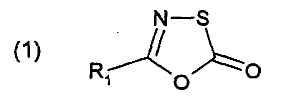 worin
- R₁: nicht substituiertes oder durch Halogen, -CN, -NO₂, -C=O, -C=S, -NR₂, -OR₃, -SR₄, -SO₂R₅, -COOR₆, mit einem 1,3,4-Oxathiazol-2-on-Rest substituiertes C₁-C₁₆-Alkyl, C₂-C₁₆-Alkenyl, C₅-C₈-Cycloalkyl; nicht substituiertes oder durch ein oder mehrere C₁-C₅-Alkyl, C₆-C₁₀-Aryl, Halogen, Hydroxy, Acyl, -CN, -CF₃, -NO₂, -NR₂, -OR₃, -SR₄, -SO₃H, -SO₂R₅, -COOR₆ oder einem 1,3,4-Oxathiazol-2-on-Rest substituiertes C₆-C₁₀-Aryl; einen 5- oder 6-gliedrigen heterocyclischen Rest;
- R₂ und R₃: unabhängig voneinander Wasserstoff; C₁-C₅-Alkyl; C₆-C₁₀-Aryl, oder Acyl;
- R₄: Wasserstoff; C₁-C₅-Alkyl; oder C₆-C₁₀-Aryl
- R₅: C₁-C₅-Alkyl; oder C₆-C₁₀-Aryl;
- R₆: Wasserstoff; C₁-C₅-Alkyl; oder C₆-C₁₀-Aryl
bedeuten.

C₁-C₁₆-Alkyl sind geradkettige oder verzweigte Alkylreste wie z.B. Methyl, Ethyl, n-Propyl, Isopropyl, n-Butyl, sek.Butyl, tert.Butyl, Amyl, Isoamyl oder tert.Amyl, Heptyl, Octyl, Isooctyl, Nonyl, Decyl, Undecyl, Dodecyl, Tetradecyl, Pentadecyl oder Hexadecyl.

C₂-C₁₆-Alkenyl bedeutet z.B. Allyl, Methallyl, Isopropenyl, 2-Butenyl, 3-Butenyl, Isobutenyl, n-Penta-2,4-dienyl, 3-Methyl-but-2-enyl, n-Oct-2-enyl, n-Dodec-2-enyl, 3,6,8-Decatrienyl oder iso-Dodecenyl.

C₆-C₁₀-Aryl kann ein mono- oder bicyclischer aromatischer Rest, wie z.B. Phenyl oder Naphthyl sein.

C₅-C₈-Cycloalkyl kann Cyclopentyl, Cycloheptyl, Cyclooctyl oder vorzugsweise Cyclohexyl sein.

Beispiele für 6-gliedrige heterocyclische Reste sind Pyranyl, Pyridyl, Pyrazinyl, Pyrimidinyl, Pyridazinyl, Piperazinyl, Indolinyl oder Morpholinyl.

Beispiele für 5-gliedriege heterocyclische Reste sind Thienyl, Furyl, 2H-Pyrrolyl, Pyrrolyl, Imidazolyl, Pyrazolyl, Isoxazolyl, Furazanyl, Pyrrolidinyl, Pyrrolinyl, Imidazolidinyl, Pyrazolidinyl oder Pyrazolinyl.

Die 5- oder 6-gliedrigen heterocyclischen Reste können unsubstituiert oder durch Halogen, wie Chlor oder Brom, Nitro, C₁-C₅-Alkyl oder C₁-C₅-Alkoxy substituiert sein.

Vorzugsweise werden erfindungsgemäss Verbindungen der Formel (1) eingesetzt, worin
- R₁: nicht substituiertes oder durch Halogen, -CN, -NO₂, -C=O, -C=S, -NR₂, -OR₃, -SR₄, -SO₂R₅, -COOR₆, mit einem 1,3,4-Oxathiazol-2-on-Rest substituiertes C₁-C₁₆-Alkyl; oder nicht substituiertes oder durch ein oder mehrere C₁-C₅-Alkyl, C₆-C₁₀-Aryl, Halogen, Hydroxy, Acyl, -CN, -CF₃, -NO₂, -NR₂, -OR₃, -SR₄, -SO₃H, -SO₂R₅, -COOR₆ oder einem 1,3,4-Oxathiazol-2-on-Rest substituiertes C₆-C₁₀-Aryl; bedeutet
und
- R₂, R₃, R₄, R₅, und R₆: die in Formel (1)angegebene Bedeutung haben.

Ganz besonders bevorzugt sind Verbindungen der Formel (1), worin
- R₁: C₁-C₁₆-Alkyl; insbesondere C₁-C₁₂-Alkyl und ganz besonders Methyl oder Ethyl bedeutet.

Weiterhin werden erfindungsgemäss vorzugsweise Oxathiazolone der Formel 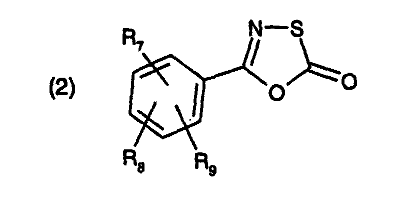 verwendet, worin
- R₇, R₈ und R₉: unabhängig voneinander Wasserstoff; Halogen; Hydroxy; oder C₁-C₅-Alkyl;
bedeuten.

Beispielhafte erfindungsgemäss einsetzbare Verbindungen entsprechen den Formeln 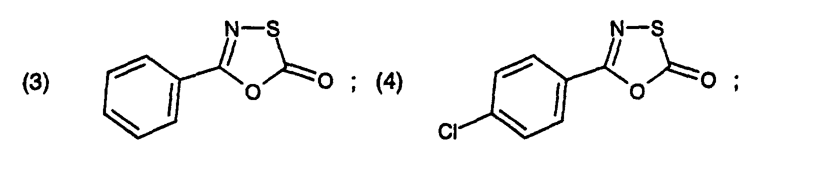 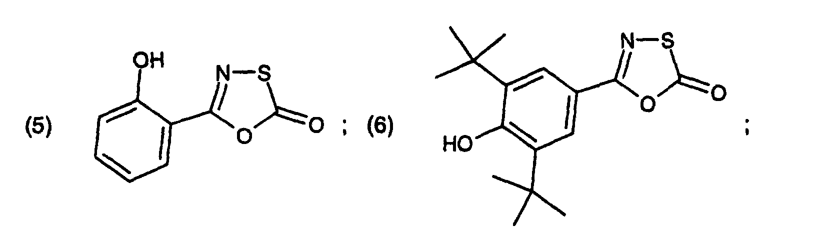 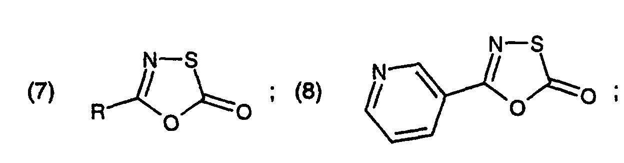 oder

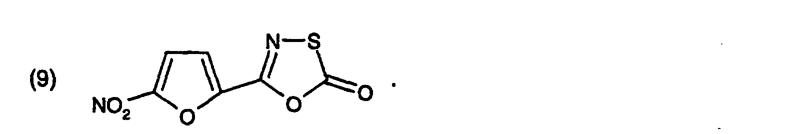

In Formel (7) bedeutet R = -CH₃; oder -(CH₂)₁₀CH₃.

Die Verbindungen der Formeln (1) bis (8) sind literaturbekannt. Die Vebindung der Formel (9) stellt eine neue Verbindung dar.

Die Herstellung der erfindungsgemäss eingesetzten Oxanilide erfolgt nach bekannten Verfahren durch Umsetzung des Carbonsäureamids der Formel 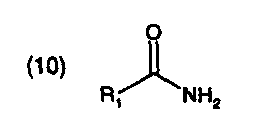 mit Chlorcarbonylsulfensäurechlorid, entsprechend der Formel 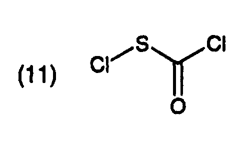 nach folgendem Schema: 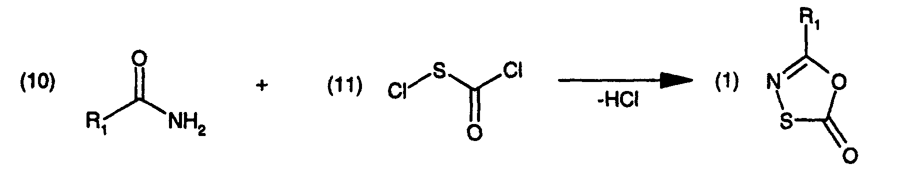 R₁ hat dabei die in Formel (1) angegebene Bedeutung.

Die Ausgangsstoffe entsprechend Formel (7) sind bekannt. Chlorcarbonylsulfensäurechlorid (Verbindung der Formel (11)) ist ein bekanntes Reagenz der organischen Chemie.

Das Herstellungsverfahren wird vorzugsweise ohne, kann aber auch in Gegenwart eines geeigneten Säureakzeptors durchgeführt werden. Als solche kommen alle üblichen organischen oder anorganischen Basen in Betracht. Hierzu gehören beispielsweise Erdalkalimetall- oder Alkalimetallhydride , -hydroxide, -amide, -alkoholate, -acetate, -carbonate oder -hydrogencarbonate, wie beispielsweise Natriumhydrid, Natriumamid, Natriummethylat, Natriumethylat, Kalium-tert.butylat, Natriumhydroxid, Kaliumhydroxid, Ammoniumhydroxid, Natriumacetat, Kaliumacetat, Calciumacetat, Amoniumacetat, Natriumcarbonat, Kaliumcarbonat, Natrium-, Kaliumcarbonat oder -hydrogencarbonat, Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin oder N,N-Dimethylaminopyridin.

Das Verfahren wird vorzugsweise in Gegenwart eines Lösungsmittels durchgeführt. Es kommen dafür alle üblichen organischen Lösungsmittel in Betracht. Hierzu gehören insbesondere aliphatische, alicyclische oder aromatische, gegebenenfalls halogenierte Kohlenwasserstoffe, wie z.B. Benzol, Toluol, Xylol, Chlorbenzol, Dichlorbenzol, Petrolether, Hexan, Cyclohexan, Dichlormethan, Chloroform, Tetrachlorkohlenstoff, Ether, wie Dimethylether, Diethylether, Dioxan, Tetrahydrofuran oder Ethylenglykoldimethylether, Ketone, wie Aceton, Butanon oder Methyl-isobutyl-keton, Nitrile, wie Acetonitril, Propionitril oder Benzonitril; Amide, wie N,N-Dimethylformamid, N,N-Dimethylacetamid, N,N-Methylformanilid, N-Methylpyrrolidon oder Hexamethylphosphorsäuretriamid; Ester wie Essigsäuremethyl- oder ethylester, Sulfoxide, wie Dimethylsulfoxid, bevorzugt Kohlenwasserstoffe. Besonders bevorzugt sind Toluol oder Dioxan.

Die Reaktionstemperaturen können in einem weiten Bereich variiert werden. Im allgemeinen arbeitet man bei Temperaturen von -10 und +180°C, vorzugsweise bei Temperaturen zwischen 20 und 130°C.

Das Verfahren wird gewöhnlich unter Normaldruck durchgeführt, kann aber auch bei erhöhtem oder vermindertem Druck durchgeführt werden.

Zur Durchführung des Verfahrens setzt man je Mol an Carbonsäureamid der Formel (10) im allgemeinen 1 bis 10 Mol, vorzugsweise 1,0 bis 3 Mol an Chlorcarbonylsulfensäurechlorid ein.

Die erfindungsgemäss eingesetzten Oxathiazolone zeigen ausgeprägte antimikrobielle Wirkung, insbesondere gegen pathogene grampositive und gramnegative Bakterien sowie gegen Bakterien der Hautflora, wie z.B. Corynebacterium xerosis (Bakterien, die Körpergeruch verursachen), ausserdem gegen Hefen und Schimmelpilze. Sie eignen sich daher insbesondere zur Desinfektion der Haut und Schleimhäute sowie Hautanhangsgebilden (Haare), ganz besonders zur Hände- und Wunddesinfektion.

Sie sind daher geeignet als antimikrobielle Wirksubstanz in Körperpflegemitteln, wie z.B. Shampoos, Badezusätzen, Haarpflegemitteln, flüssigen und festen Seifen (auf Basis synthetischer Tenside und Salze von gesättigten und/oder ungesättigten Fettsäuren), Lotionen und Cremes, Deodorantien, anderen wässrigen oder alkoholischen Lösungen, z.B. Reinigungslösungen für die Haut, feuchte Reinigungstücher, Ölen oder Pudern.

Einen weiteren Erfindungsgegenstand bildet daher ein Körperpflegemittel, enthaltend mindestens eine Verbindung der Formel (1) sowie kosmetisch verträgliche Träger- oder Hilfsstoffe.

Das erfindungsgemässe Körperpflegemittel enthält 0,01 bis 15, vorzugsweise 0,5 bis 10 Gew.-%, bezogen auf das Gesamtgewicht der Zusammensetzung, der Oxathiazolon-Verbindung der Formel (1) und kosmetisch verträgliche Hilfsstoffe.

Je nachdem, in welcher Form das Körperpflegemittel vorliegt, weist es neben der Oxathiazolon-Verbindung der Formel (1) noch weitere Bestandteile auf, wie z.B. Sequestriermittel, Farbstoffe, Parfümöle, Verdickungs- bzw. Festigungs (Konsistenzregler)-mittel, Emmollients, UV-Absorber, Hautschutzmittel, Antioxidantien, die mechanischen Eigenschaften verbessernde Additive wie Dicarbonsäuren und/oder Al-, Zn-, Ca-, Mg-Salze von C₁₄-C₂₂-Fettsäuren und gegebenenfalls Konservierungsmittel.

Das erfindungsgemässe Körperpflegemittel kann als Wasser-in-Öl- oder Öl-in-Wasser-Emulsion, als alkoholische oder alkoholhaltige Formulierung, als vesikulare Dispersion eines ionischen oder nichtionischen amphiphilen Lipids, als Gel, fester Stift oder als AerosolFormulierung formuliert werden.

Als Wasser-in-Öl- oder Öl-in-Wasser-Emulsion enthält der kosmetisch verträgliche Hilfs stoff vorzugsweise 5 bis 50% einer Ölphase, 5 bis 20% eines Emulgators und 30 bis 90% Wasser. Die Ölphase kann dabei irgendein für kosmetische Formulierungen geeignetes Öl enthalten, wie z.B. ein oder mehrere Kohlenwasserstofföle, ein Wachs, ein natürliches Öl, ein Silikon-Öl, einen Fettsäureester oder einen Fettalkohol. Bevorzugte Mono- oder Polyole sind Ethanol, Isopropanol, Propylenglykol, Hexylenglycol, Glycerin und Sorbitol.

Erfindungsgemässe kosmetische Formulierungen beinhalten die verschiedensten kosmetische Mittel. Insbesondere kommen z.B. die folgenden Mittel in Betracht:
- Mittel zur Hautpflege, wie z.B. Hautwasch- und Reinigungsmittel in Form von stückförmigen oder flüssigen Seifen, Syndets oder Waschpasten,
- Badepräparate, wie z.B. flüssige (Schaumbäder, Milche, Duschpräparate) oder feste Badepräparate, wie z.B. Badetabletten und Badesalze;
- Hautpflegemittel, wie z.B. Hautemulsionen, Mehrfachemulsionen oder Hautöle;
- Dekorative Körperpflegemittel, wie z.B. Gesichts-Make-ups in Form von Tages- oder Pudercremes, Gesichtspuder (lose und gepresst), Rouge oder Creme-Make-ups, Augenpflegemittel, wie z.B. Lidschattenpräparate, Wimperntusche, Eyeliner, Augencremes oder Eye-Fix-Cremes; Lippenpflegemittel, wie z.B. Lippenstift, Lip Gloss, Lippenkonturstift, Nagelpflegemittel, wie Nagellack, Nagellackentferner, Nagelhärter, oder Nagelhautentfemer;
- Intimpflegemittel, wie z.B. Intim-Waschlotionen oder Intimsprays;
- Fusspflegemittel, wie z.B. Fussbäder, Fusspuder, Fusscremes bzw. Fussbalsame, spezielle Deomittel und Antitranspirantien oder hornhautbeseitigende Mittel;
- Lichtschutzmittel, wie Sonnenmilche, -lotionen, -cremes, -öle, Sun-blockers oder Tropicals, Vorbräunungspräparate oder After-sun-Präparate;
- Hautbräunungsmittel, wie z.B. Selbstbräunungscremes;
- Depigmentierungsmittel, wie z.B. Präparate zur Hautbleichung oder Mittel zur Hautaufhellung;
- Insektenabweisende Mittel ("Repellents"), wie z.B. Insektenöle, -lotionen, -sprays, oder -stifte;
- Deodorantien, wie Deosprays, Pumpsprays, Deogele, -stifte oder -roller;
- Antitranspirantien, wie z.B. Antitranspirantstifte, -cremes oder -roller;
- Mittel zur Reinigung und Pflege von unreiner Haut, wie z.B. Syndets (fest oder flüssig), Peeling- oder Scrubb-Präparate oder Peeling-Masken;
- Haarentfernungsmittel in chemischer Form (Depilation), wie z.B. Haarentfernungspulver, flüssige Enthaarungsmittel, cremige oder pastöse Enthaarungsmittel, Enthaarungsmittel in Gelform oder Aerosolschäume;
- Rasiermittel, wie z.B. Rasierseife, schäumende Rasiercremes, nichtschäumende Rasiercremes, -schäume, -gele, Preshave-Präparate für die Trockenrasur, Aftershaves oder Aftershave-Lotionen;
- Duftmittel, wie z.B. Duftwässer (Eau de Cologne, Eau de Toilette, Eau de Parfum, Parfum de Toilette, Parfüm), Parfümöle oder Parfümcremes;
- Mittel zur Zahn-, Zahnersatz- und Mundpflege, wie z.B. Zahncremes, Gel-Zahncremes, Zahnpulver, Mundwasserkonzentrate, Anti-Plaque-Mundspülungen, Prothesenreiniger oder Prothesenhaftmittel;
- Kosmetische Mittel zur Haarbehandlung, wie z.B. Haarwaschmittel in Form von Schampoos, Haarkonditioniermittel, Haarpflegemittel, wie z.B. Vorbehandlungsmittel, Haarwasser, Frisiercremes, Frisiergele, Pomaden, Haarspülungen, Kurpackungen, Intensivhaarkuren, Mittel zur Haarverformung, wie z.B. Wellmittel zur Herstellung von Dauerwellen (Heisswelle, Mildwelle, Kaltwelle), Haarglättungspräparate, flüssige Haarfestiger, Haarschäume, Haarsprays, Blondiermittel, wie. z.B. Wasserstoffperoxidlösungen, aufhellende Schampoos, Blondiercremes, Blondierpulver, Blondierbreie oder -öle, temporäre, semitemporäre oder permanente Haarfärbemittel, Präparate mit selbstoxidierenden Farbstoffen, oder natürliche Haarfärbemittel, wie Henna oder Kamille.

Eine antimikrobielle Seife hat z.B. folgende Zusammensetzung:
0,01 bis 5 Gew.% der Verbindung der Formel (1)
0,3 bis 1 Gew.% Titandioxid,
1 bis 10 Gew.% Stearinsäure
ad 100% Seifengrundlage, wie z.B. die Natriumsalze der Talgfett- und Kokosfettsäure oder Glycerine.

Ein Schampoo hat z.B. die folgende Zusammensetzung:
0,01 bis 5 Gew.% der Verbindung der Formel (1),
12,0 Gew.% Natrium-Laureth-2-sulfat,
4,0 Gew.% Cocamidopropylbetain,
3,0 Gew.% NaCI und
Wasser ad 100%.

Ein Deodorant hat z.B. die folgende Zusammensetzung:
0,01 bis 5 Gew.% der Verbindung der Formel (1),
60 Gew.% Ethanol,
0,3 Gew.% Parfümöl, und
Wasser ad 100 %.

Einen weiteren Erfindungsgegenstand ist eine orale Zusammensetzung, enthaltend 0,01 bis 15 Gew.-%, bezogen auf das Gesamtgewicht der Zusammensetzung, der Verbindung der Formel (1) und oral verträgliche Hilfsstoffe.

Beispiel für eine orale Zusammensetzung:
10 Gew.% Sorbitol,
10 Gew.% Glycerin,
15 Gew.% Ethanol,
15 Gew.% Propylenglycol,
0,5 Gew.% Natriumlaurylsulfat,
0,25 Gew.% Natriummethylcocyltaurat,
0,25 Gew.% Polyoxypropylen/Polyoxyethylen-Blockcopolymer,
0,10 Gew.% Pfefferminzgeschmacksstoff,
0,1 bis 0,5 Gew.% einer Verbindung der Formel (1), und
48,6 Gew.% Wasser.

Die erfindungsgemässe orale Zusammensetzung kann z.B. in Form eines Gels, einer Paste, einer Creme oder einer wässrigen Zubereitung (Mundwasser) vorliegen.

Weiterhin kann die erfindungsgemässe orale Zusammensetzung Verbindungen enthalten, die Fluoridionen freisetzen, die gegen die Bildung von Karies wirksam sind, z.B. anorganische Fluoridsalze, wie z.B. Natrium-, Kalium-, Ammonium- oder Calciumfluorid oder organische Fluoridsalze, wie z.B. Aminfluoride, die unter dem Handelsnamen Olafluor bekannt sind.

Bei den textilen Fasermaterialien handelt es sich um ungefärbte und gefärbte oder bedruckte Fasermaterialien z.B. aus Seide, Wolle, Polyamid oder Polyurethanen, und insbesondere cellulosehaltige Fasermaterialien aller Art. Solche Fasermaterialien sind beispielsweise natürliche Cellulosefasern, wie Baumwolle, Leinen, Jute und Hanf, sowie Zellstoff und regenerierte Cellulose. Bevorzugte geeignete textile Fasermaterialien sind aus Baumwolle.

Weiterhin finden die Oxathiazolone der Formel (1) Verwendung in Wasch- und Reinigungsformulierungen, wie z.B. in Flüssig- und Pulverwaschmitteln oder Weichspülern.

Die Oxathiazolone können insbesondere auch in Haushalts- und Allzweckreinigem zur Reinigung und Desinfektion von harten Oberflächen eingesetzt werden.

Ein Reinigungsmittel hat z.B. folgende Zusammensetzung:
0,01 bis 5 % der Verbindung der Formel (1)
3,0 % Octylalkohol 4EO
1,3 % Fettalkohol C₈-C₁₀-Polyglucosid
3,0 % Isopropanol
ad 100 % Wasser.

Weiterhin eignen sich die erfindungsgemässen Verbindungen zum Schutz von kosmetischen Produkten und Haushaltsprodukten vor mikrobieller Verderbnis.

Die folgenden Beispiele dienen der Veranschaulichung der Erfindung, ohne diese auf die Beispiele zu beschränken.

### Beispiel 1: Herstellung der Verbindung der Formel (101):

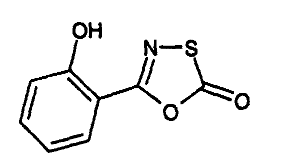

6,5 g (0,05 Mol) Chlorcarbonylsulfenylchlorid, gelöst in 60 ml Dioxan, werden zu 4,0 g (0,029 Mol) Salicylamid gegeben. Das Reaktionsgemisch wird 3 Stunden unter Rückfluss erhitzt. Es entsteht eine klare Lösung. Da das TLC (="Thin Layer Chromatography") eine kleine Menge an nicht reagiertem Ausgangsmaterial anzeigt, werden weitere 0,5 ml Chlorcarbonylsulfenylchlorid hinzugefügt und unter Rühren für eine weitere Stunde unter Rückfluss erhitzt.
Anschliessend wird das Lösungsmittel unter Vakuum entfernt und der Rückstand säulenchromatographisch (5% Ethylacetat in Petrolether) gereinigt.
Ausbeute: 3,8 g (68% d.Th.)

### Beispiel 2: Herstellung der Verbindung der Formel (102):

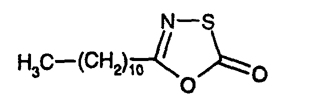

5,0 g (0,025 Mol) Lauroylamid werden in 30 ml Dioxan durch Erhitzten auf 110°C gelöst. Zu dieser Lösung werden 5,0 g (0,038 Mol) Chlorcarbonylsulfenylchlorid hinzugefügt und für weitere 5 bis 6 Stunden erhitzt. Die Reaktion wird durch TLC kontrolliert. Nach Vervollständigung der Reaktion wird das Reaktionsgemisch, das noch einige suspendierte Partikel enthält, gefiltert. Das Filtrat wird zur festen Verbindung eingeengt, die säulenchromatographisch gereinigt wird (Petrolether/Ethylacetatgemisch).
Ausbeute: 3,87 g (60% d.Th.)

### Beispiel 3: Herstellung der Verbindung der Formel (103):

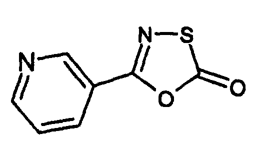

4,5 g (0,036 Mol) Nicotinamid werden in 20 ml Toluol unter Rückfluss erhitzt. Zu dieser heissen Lösung werden 6,5 g (0,05 Mol) Chlorcarbonylsulfenylchlorid, gelöst in 15 ml Toluol, hinzugefügt. Unter Rückfluss wird für weitere 6 Stunden erhitzt. Nach Beendigung der Reaktion wird das Reaktionsgemisch mit NaHCO₃ gewaschen. Der feste Teil wird getrennt und säulenchromatographisch (Petrolether/Ethylaacetat-Mischung) gereinigt.
Ausbeute: 1,5 g (23% d.Th.).

### Beispiel 4: Herstellung der Verbindung der Formel (104):

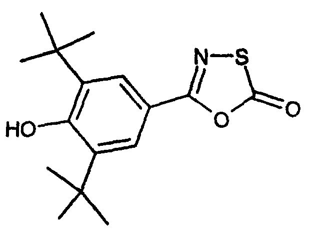

4,35 g (0,033 Mol) Chlorcarbonylsulfenylchlorid, gelöst in 10 ml Dioxan werden zu 5,0 g (0,02 Mol) 3,5-di-tert-butyl-4-hydroxybenzamid in 30 ml Dioxan unter Rückflusstemperatur tropfenweise hinzugefügt. Die Reaktion startet sofort. Das Lösungsmittel wird unter reduziertem Druck entfernt und der Rückstand durch Umkristallisation aus einem Petrolether-Ethylacetatgemisch gereinigt.
Ausbeute: 2,95 g (48% d.Th.)

### Beispiel 5: Herstellung der Verbindung der Formel (105):

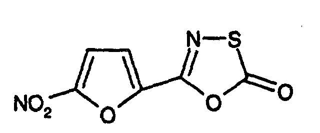

6,0 g (0,038 Mol) 5-Nitro-5-Furansäureamid werden in 30 ml Dioxan durch Erhitzen auf 80°C gelöst. Zu dieser heissen Lösung werden 7,55 g (0,057 Mol) Chlorcarbonylsulfensäurechlorid in 10 ml Toluol gelöst und unter Rückfluss 3 Stunden erhitzt. Nach Beendigung der Reaktion wird das Reaktionsgemisch unter reduziertem Druck eingeengt und der Rückstand durch Umkristallisieren aus einem Petrolether-Ethylacetatgemisch gereinigt.
Ausbeute: 2,6 g (31,6 % d. Th.)

### Beispiel 6: Herstellung der Verbindung der Formel (106):

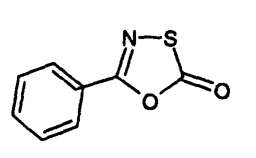

Ein Gemisch aus 60,5 g (0,5 mol) Benzamid, 65,5 g (0,5 Mol) Chlorcarbonylsulfenylchlorid und 250 ml Toluol werden unter Rühren vorsichtig auf 60 bis 90°C erhitzt. Gegen Ende der HCl-Entwicklung nach ca. 2 Stunden wird das Reaktionsgemisch für eine Stunde auf 100 bis 110°C erhitzt. Nach Entfernung des Lösungsmittels wird der feste Rückstand aus Petrolether/Ethylacetat umkristallisiert.
Ausbeute: 44,9 g (50%).

### Beispiel 7: Herstellung eines Flüssigwaschmittels:

0,01-5% der Verbindung der Formel (106)
15.0% PEG-7 C₁₄-C₁₅-Alkoholether
10.0% Natriumdodecylbenzolsulfonat
10.0% Propyleneglycol
3.0% Natriumcitrat
ad 100% deionisiertes Wasser

Die Verbindung der Formel (106) wird in C₁₄-C₁₅-Alkoholether bei 50°C gelöst. Propylenglycol und Natriumdodecylbenzolsulfonat werden zugegeben und homogen gerührt. Nach Abkühlen auf 22°C werden Natriumcitrat und Wasser zugegeben.

### Beispiel 8: Herstellung eines Weichspülmittels:

0-5% der Verbindung der Formel (106)
4,0% der Verbindung der Formel 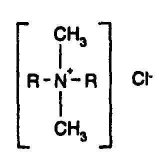 R = aliphatischer Rest der Talgfettsäure bedeutet,
0,5% Pareth-25-7, und
ad 100% deionisiertes Wasser

Die Verbindung der Formel (106) wird in Quaternium 18 bei 40°C gelöst, Pareth-25-7 und Wasser werden zugegeben und gerührt, bis eine homogene Mischung entsteht.

### Beispiel 9: Herstellung eines Geschirrspülmittels

0,01-5% der Verbindung der Formel (106)
7,0% Natriumlaurylsulfat
7,0% Natriummyrethsulfat
4,0% Laurylglucosid
1,1% Cocobetain
5,0% Ethanol
1,0% Citronensäure
ad 100% deionisiertes Wasser

Die Verbindung der Formel (106) wird in Ethanol gelöst. Die Tenside (Natriumlaurylsulfat, Natriummyrethsulfat, Laurylglucosid und Cocobetain) werden vorgelöst in Wasser zugegeben und bei 40°C homogen gerührt. Citronensäure und Wasser werden der Lösung bei 22°C zugegeben.

### Beispiel 10: Herstellung einer Flüssigseife

0,01-5%der Verbindung der Formel (106)
10.0% Natriumlaureth-2-sulfate
3.0% Cocamidopropylbetain
2.0% Laurylglucosid
1.0% Glycoldistearat
1.0% Natriumchlorid
ad 100% deionisiertes Wasser

Die Verbindung der Formel (106) wird in Cocamidopropylbetain und Laurylglucosid bei 50°C unter Rühren solubilisiert. Natriumlaureth-2-sulfat und Glykoldistearat werden zugegeben und bei 50°C homogen gerührt. Nach vollständigem Auflösen von Glykoldistearat wird unter Rühren auf Raumtemperatur abkekühlt, Wasser zugegeben und mit Natriumchlorid die Viskosität eingestellt.

### Beispiel 11: Herstellung eines Oberflächendesinfektionsmittels

0,01 bis 5 % der Verbindung der Formel (106)
3,0 % Octylalkohol 4EO
1,3 % Fettalkohol C₈-C₁₀-Polyglucosid
3,0 % Isopropanol
ad 100 % Wasser.

Die Verbindung der Formel (106) wird in Isopropanol gelöst, Octylalkohol 4EO und Fettalkohol C₈-C₁₀-Polyglucosid werden der Lösung zugegeben und bis zur Homogenität gerührt. Der pH-Wert wird mit Ethanolamin eingestellt und die Formulierung wird mit Wasser auf 100% gestellt.

### Beispiel 12: Bestimmung der antimikrobiellen Aktivität im Agardiffusionstest

| | |
|---|---|
| Medium | Casein-Sojamehlpepton Agar ( Merck) * Sabouraud 4% Glucose-Agar (Merck) |
| | |
| Verdünnungsmedium | sterile 0.85% NaCl-Lösung |
| | |
| Testkeime | Staphylococcus aureus ATCC 9144 Escherichia coli NCTC 8196 Candida albicans ATCC 10231 * Aspergillus niger ATCC 6275 |
| | |
| Inkubation der Vorkulturen | 24 Stunden bei 37°C * 5-7 Tage bei 28°C |
| | |
| Inkubation der Testplatten | 24 Stunden bei 37°C * 4 Tage bei 28°C |

Nach Inkubation der Bakterien-Vorkulturen werden diese mit Verdünnungsmedium 1:100 verdünnt.
3,5 ml dieser Verdünnung der Bakterien werden in 500 ml Agar gegeben und von diesem Keim-haltigen Agar werden Platten mit etwa 15 ml Medium gegossen.
Die Testsubstanzen werden in einem geeigneten Lösungsmittel gelöst in Endkonzentrationen von 1-3%.
Nach Erkalten und Verfestigung der Agarplatten werden Löcher in den Agar gestanzt (Durchmesser 10mm) und je 100 µl der Testsubstanz-Lösungen eingefüllt.
Nach Inkubation der Platten werden die Durchmesser der wachstumsfreien Hemmhöfe im Agar bestimmt.

Die Testergebnisse für die Verbindung der Formel (107) 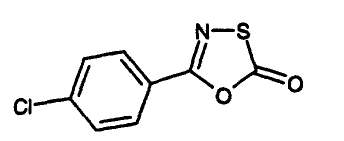 sind in Tabelle 1 aufgeführt:

**Tabelle 1:**

| | Hemmhofdurchmesser (mm) | |
|---|---|---|
| Mikroorganismen | 2% in Ethanol | 1% in Ethanol |
| Staphylococcus aureus ATCC 9144 | 5/5 | 4/4 |
| Escherichia coli NCTC 8196 | 1/1 | 1/1 |
| Candida albicans ATCC 10231 | 4/4 | 3/3 |
| Aspergillus niger ATCC 6275 | 0/0 | 0/0 |

Die Testergebnisse für die Verbindung der Formel (108) 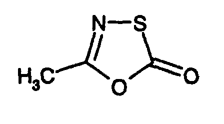 sind in Tabelle 2 aufgeführt.

**Tabelle 2:**

| | Hemmhofdurchmesser (mm) | |
|---|---|---|
| Mikroorganismen | 2% in Ethanol | 1% in Ethanol |
| Staphylococcus aureus ATCC 9144 | 20/20 | 12/12 |
| Escherichia coli NCTC 8196 | 3/2 | 1/1 |
| Candida albicans ATCC 10231 | 10/10 | 8/8 |
| Aspergillus niger ATCC 6275 | 12 / 12 | 4 / 5 |

Die Testergebnisse für die Verbindung der Formel (105) sind in Tabelle 3 aufgeführt:

**Tabelle 3:**

| | Hemmhofdurchmesser (mm) | | | |
|---|---|---|---|---|
| Mikroorganismen | Staphylococcus aureus ATCC 9144 | Escherichia coli NCTC 8196 | Pseudomonas aeruginosa ATCC 15'442 | Candida albicans ATCC 10'231 |
| Verbindung der Formel (105): | | | | |
| 3,0% in THF | 11/11 | 6/6 | 6/6 | 9/9 |
| 1,0% in THF | 11/11 | 6/6 | 5/5 | 8/8 |
| 0,5% in THF | 10/10 | 3/3 | 1/1 | 4/4 |
| 0,1% in THF | 4/4 | 1/1 | 0/0 | 3/3 |

### Bestimmung der minimalen Hemmkonzentration (MHK) im Agarinkorporationstest (MHK-Test)

| | |
|---|---|
| Medium | Casein-Sojamehlpepton Agar (Merck) *Sabouraud 4% Glucose-Agar (Merck) |
| | |
| Verdünnungsmedium | sterile 0.85% NaCl-Lösung |
| | |
| Testkeime | Staphylococcus aureus ATCC 9144 Staphylococcus epidermidis ATCC 12228 Corynebacterium xerosis ATCC 373 Escherichia coli NCTC 8196 Pseudomonas aeruginosa CIP A-22 Candida albicans ATCC 10231 *Aspergillus niger ATCC 6275 |
| | |
| Inkubation | 24 Stunden bei 37°C *3 Tage bei 28°C |
| | |
| Testlösung | Von allen Testsubstanzen werden 1%ige Stammlösungen in einem geeigneten Lösungsmittel hergestellt und in Verdünnungsreihen auf Endkonzentrationen von 1000ppm bis 10ppm verdünnt. |

### Testprinzip:

0,3 ml der jeweiligen Verdünnungsstufe werden mit 15 ml noch flüssigem Nährmedium gemischt. Nach Erstarren des Nährbodens werden von den Teststämmen punktweise je 10µl der folgenden Keimverdünnung in 0.85% NaCl-Lösung auf das Agarmedium aufgetragen:

| | |
|---|---|
| Staphylococcus aureus ATCC 9144 | 1:100-Verdünnung |
| Staphylococcus epidermidis ATCC 12228 | 1:100-Verdünnung |
| Corynebacterium xerosis ATCC 373 | 1:100-Verdünnung |
| Escherichia coli NCTC 8196 | 1:1000-Verdünnung |
| Pseudomonas aeruginosa CIP A-22 | 1:1000-Verdünnung |
| Candida albicans ATCC 10'231 | 1:10-Verdünnung |
| Aspergillus niger ATCC 6275 | 1:10-Verdünnung |

Die Platten werden während 24 Stunden bei 37°C bebrütet ( A.niger 3 Tage bei 28°C) und anschliessend die höchste Verdünnung der Testsubstanz bestimmt, bei der gerade kein Wachstum mehr beobachtet wurde (entspricht MHK).

Die Testergebnisse sind in Tabelle 4 aufgeführt.

**Tabelle 4:**

| Keime | Verbindung der Formel (106) (Lsg. in DMSO) | Verbindung der Formel (103) (Lsg. in EtOH) | Verbindung der Formel (101) (Lsg. in EtOH) |
|---|---|---|---|
| S. aureus | 10 | 100 | 100 |
| S. epidermidis | 10 | 100 | 100 |
| C. xerosis | 10 | 200 | 250 |
| E. coli | 100 | 600 | 1000 |
| P. aeruginosa | 1000 | 600 | 1000 |
| C. albicans | 100 | 100 | 100 |
| A. niger | 250 | 600 | 100 |
| MIC Konzentrationen in ppm | | | |

Die Ergebnisse zeigen eine starke antimikrobielle Aktivität der Testsubstanzen gegen grampostive und gram-negative Bakterien sowie Pilze und Hefen.

## Patentansprüche

1. Verwendung der Verbindungen der Formel 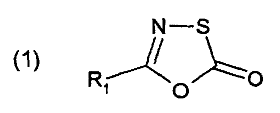 worin
R₁ nicht substituiertes oder durch Halogen, -CN, -NO₂, -C=O, -C=S, -NR₂, -OR₃, -SR₄, -SO₂R₅, -COOR₆, mit einem 1,3,4-Oxathiazol-2-on-Rest substituiertes C₁-C₁₆-Alkyl, C₂-C₁₆-Alkenyl, C₅-C₈-Cycloalkyl; nicht substituiertes oder durch ein oder mehrere C₁-C₅-Alkyl, C₆-C₁₀-Aryl, Halogen, Hydroxy, Acyl, -CN, -CF₃, -NO₂, -NR₂, -OR₃, -SR₄, -SO₃H, -SO₂R₅, -COOR₆ oder einem 1,3,4-Oxathiazol-2-on-Rest substituiertes C₆-C₁₀-Aryl; einen 5- oder 6-gliedrigen heterocyclischen Rest;
R₂ und R₃ unabhängig voneinander Wasserstoff; C₁-C₅-Alkyl; C₆-C₁₀-Aryl, oder Acyl;
R₄ Wasserstoff; C₁-C₅-Alkyl; oder C₆-C₁₀-Aryl
R₅ C₁-C₅-Alkyl; oder C₆-C₁₀-Aryl;
R₆ Wasserstoff; C₁-C₅-Alkyl; oder C₆-C₁₀-Aryl
bedeuten, zur antimikrobiellen Behandlung von Oberflächen, ausgewählt aus Haut, Schleimhäuten, Haaren und textilen Fasermaterialien.

2. Verwendung nach Anspruch 1, **dadurch gekennzeichnet, dass** in den Verbindungen der Formel (1)
R₁ nicht substituiertes oder durch Halogen, -CN, -NO₂, -C=O, -C=S, -NR₂, -OR₃, -SR₄, -SO₂R₅, -COOR₆, mit einem 1,3,4-Oxathiazol-2-on-Rest substituiertes C₁-C₁₆-Alkyl; oder nicht substituiertes oder durch ein oder mehrere C₁-C₅-Alkyl, Hydroxy, Halogen oder einem 1,3,4-Oxathiazol-2-on-Rest substituiertes Phenyl;
bedeutet, und
R₂, R₃, R₄, R₅, und R₆ die in Anspruch 1 angegebene Bedeutung haben.

3. Verwendung nach Anspruch 1 oder 2, **dadurch gekennzeichnet, dass**
R₁ C₁-C₁₆-Alkyl;
bedeutet.

4. Verwendung nach Anspruch 1 oder 2, **dadurch gekennzeichnet, dass** Oxathiazolone der Formel 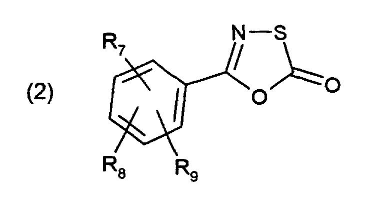 verwendet werden, worin
R₇, R₈ und R₉ unabhängig voneinander Wasserstoff; Halogen; Hydroxy; oder C₁-C₅-Alkyl; bedeuten.

5. Verwendung der Verbindung der Formel (1) gemäß Anspruch 1 in Wasch- und Reinigungsformulierungen.

6. Körperpflegemittel, enthaltend
0,01 bis 15 Gew.-%, bezogen auf das Gesamtgewicht der Zusammensetzung, der Verbindung der Formel (1) gemäß Anspruch 1 und kosmetisch verträgliche Hilfsstoffe.

7. Orale Zusammensetzung, enthaltend
0,01 bis 15 Gew.-%, bezogen auf das Gesamtgewicht der Zusammensetzung, der Verbindung der Formel (1) gemäß Anspruch 1 und oral verträgliche Hilfsstoffe.

## Claims

1. Use of a compound of formula 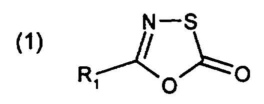 wherein
R₁ is C₁-C₁₆alkyl, C₂-C₁₆alkenyl or C₅-C₈cycloalkyl, each unsubstituted or substituted by halogen, -CN, -NO₂, -C=O, -C=S, -NR₂, -OR₃, -SR₄, -SO₂R₅, -COOR₆ or by a 1,3,4-oxathiazol-2-one radical; C₆-C₁₀aryl unsubstituted or substituted by one or more C₁-C₅alkyl, C₆-C₁₀aryl, halogen, hydroxy, acyl, -CN, -CF₃, -NO₂, -NR₂, -OR₃, -SR₄, -SO₃H, -SO₂R₅, -COOR₆ substituents or by a 1,3,4-oxathiazol-2-one radical; or a 5- or 6-membered heterocyclic radical;
R₂ and R₃ are each independently of the other hydrogen; C₁-C₅alkyl; C₆-C₁₀aryl, or acyl;
R₄ is hydrogen; C₁-C₅alkyl; or C₆-C₁₀aryl;
R₅ is C₁-C₅alkyl; or C₆-C₁₀aryl;
R₆ is hydrogen; C₁-C₅alkyl; or C₆-C₁₀aryl,
in the antimicrobial treatment of surfaces selected from skin, mucosa, hair and textile fibre materials.

2. Use according to claim 1, wherein in the compound of formula (1)
R₁ is C₁-C₁₆alkyl unsubstituted or substituted by halogen, -CN, -NO₂, -C=O, -C=S, -NR₂, -OR₃, -SR₄, -SO₂R₅, -COOR₆ or by a 1,3,4-oxathiazol-2-one radical; or phenyl unsubstituted or substituted by one or more C₁-C₅alkyl, hydroxy, halogen substituents or by a 1,3,4-oxathiazol-2-one radical;
and
R₂, R₃, R₄, R₅ and R₆ are as defined in claim 1.

3. Use according to claim 1 or 2, wherein
R₁ is C₁-C₁₆alkyl.

4. Use according to claim 1 or 2, wherein there is used an oxathiazolone of formula 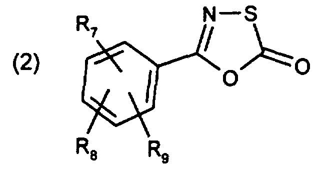 wherein
R₇, R₈ and R₉ are each independently of the others hydrogen; halogen; hydroxy; or C₁-C₅alkyl.

5. Use of a compound of formula (1) according to claim 1 in washing and cleaning formulations.

6. A personal care preparation, comprising
from 0.01 to 15 % by weight, based on the total weight of the composition, of a compound of formula (1) according to claim 1, and cosmetically tolerable adjuvants.

7. An oral composition, comprising
from 0.01 to 15 % by weight, based on the total weight of the composition, of a compound of formula (1) according to claim 1, and orally tolerable adjuvants.

## Revendications

1. Utilisation des composés de formule 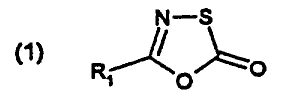 où
R₁ représente un groupe alkyle en C₁-C₁₆, alcényle en C₂-C₁₆, cycloalkyle en C₅-C₈ non substitué ou substitué par des substituants halogène, -CN, -NO₂, -C=O, -C=S, -NR₂, -OR₃, -SR₄, -SO₂R₅, -COOR₆, par un reste 1,3,4-oxathiazol-2-one ; un groupe aryle en C₆-C₁₀ non substitué ou substitué par un ou plusieurs substituants alkyle en C₁-C₅, aryle en C₆-C₁₀, halogène, hydroxy, acyle, -CN, -CF₃, -NO₂, -NR₂, -OR₃, -SR₄, -SO₃H, -SO₂R₅, -COOR₆ ou un reste 1,3,4-oxathiazol-2-one ; un reste hétérocyclique à 5 ou 6 chaînons ;
R₂ et R₃ représentent, indépendamment l'un de l'autre, un atome d'hydrogène, un groupe alkyle en C₁-C₅ ; aryle en C₆-C₁₀ ou acyle ;
R₄ représente un atome d'hydrogène ; un groupe alkyle en C₁-C₅ ; ou aryle en C₆-C₁₀ ;
R₅ représente un groupe alkyle en C₁-C₅ ; ou aryle en C₆-C₁₀ ;
R₆ représente un atome d'hydrogène ; un groupe alkyle en C₁-C₅ ; ou aryle en C₆-C₁₀,
pour le traitement anti-microbien de surfaces prises parmi la peau, les muqueuses, les cheveux et les matières fibreuses textiles.

2. Utilisation selon la revendication 1, **caractérisée en ce que** dans les composés de formule (1)
R₁ représente un groupe alkyle en C₁-C₁₆ non substitué ou substitué par des substituants halogène, -CN, -NO₂, -C=O, -C=S, -NR₂, -OR₃, -SR₄, -SO₂R₅, -COOR₆, par un reste 1,3,4-oxathiazol-2-one ; un groupe phényle non substitué ou substitué par un ou plusieurs substituants alkyle en C₁-C₅, hydroxy, halogène ou un reste 1,3,4-oxathiazol-2-one ;
et
R₂, R₃, R₄, R₅ et R₆ possèdent les significations données à la revendication (1).

3. Utilisation selon la revendication 1 ou 2, **caractérisée en ce que**
R₁ représente un groupe alkyle en C₁-C₁₆.

4. Utilisation selon la revendication 1 ou 2, **caractérisée en ce qu'**on utilise des oxathiazolones de formule 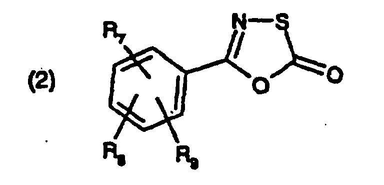 où
R₇, R₈ et R₉ représentent indépendamment les uns des autres, un atome d'hydrogène ; un atome d'halogène ; un groupe hydroxy ; ou alkyle en C₁-C₅.

5. Utilisation du composé de formule (1) selon la revendication 1 dans des formulations de lavage et nettoyage.

6. Produit de soins corporels renfermant de 0,01 à 15 % en poids, par rapport au poids total de la composition, du composé de formule (1) selon la revendication 1 et des adjuvants tolérés du point de vue cosmétique.

7. Composition orale, renfermant de 0,01 à 15 % en poids, par rapport au poids total de la composition, du composé de formule (1) selon la revendication 1 et des adjuvants oralement tolérés.
